# EUROPEAN PATENT SPECIFICATION

(11) **EP 2 609 902 B1**
(45) Date of publication and mention of the grant of the patent: **01.11.2017**
(21) Application number: 11819704.5
(22) Date of filing: 14.07.2011
(51) Int. Cl.: A61K 8/31, A61K 8/06, A61K 8/27, A61K 8/29, A61K 8/35, A61K 8/41, A61K 8/891, A61K 8/894, A61Q 17/04, A61Q 19/00

(54) **WATER-IN-OIL EMULSIFIED COSMETIC PREPARATION**
HERSTELLUNG EINES WASSER-IN-ÖL-EMULSIONSKOSMETIKUMS
PRÉPARATION COSMÉTIQUE ÉMULSIONNÉE EAU-DANS-HUILE

(30) Priority: 26.08.2010 JP 2010189709
(43) Date of publication of application: 03.07.2013
(73) Proprietor: Shiseido Company, Ltd., Chuo-ku Tokyo 104-8010 (JP)
(72) Inventor: FUKUHARA Kazuto, Yokohama-shi Kanagawa 224-8558 (JP)
(74) Representative: Ter Meer Steinmeister & Partner
(86) International application number: PCT/JP2011/066074
(87) International publication number: WO 2012/026235

(56) References cited:
- JP-A- 2007 145 722
- JP-A- 2009 524 644
- "Use of specific UV filter combinations comprising BBDAPT for cosmetic preparations", IP.COM JOURNAL, IP.COM INC., WEST HENRIETTA, NY, US, 18 June 2010 (2010-06-18), XP013138739, ISSN: 1533-0001
- "Suncare compositions using amino hydroxy benzophenone derivatives", IP.COM JOURNAL, IP.COM INC., WEST HENRIETTA, NY, US, 3 October 2007 (2007-10-03), XP013122291, ISSN: 1533-0001
- "Suncare compositions with new cosmetic raw materials (3)", IP.COM JOURNAL, IP.COM INC., WEST HENRIETTA, NY, US, 18 January 2011 (2011-01-18), XP013142054, ISSN: 1533-0001

## Description

### TECHNICAL FIELD

The present invention relates to a water-in-oil emulsified cosmetic and more specifically relates to a water-in-oil emulsified cosmetic that has a high ultraviolet protection effect and still spreads well and has a smooth texture.

### BACKGROUND ART

The intermediate wavelength UV region of wavelengths from 280 to 320 nm of UV light from the sun causes scarlet patches called sunburn on skin, and in severe cases these may be accompanied by bubbles as in burn injuries. Wavelengths in the long UV region from 320 to 400 nm darken the skin, and it is known that repeated exposure over long periods of time to either of these wavelength regions accelerates skin aging.

To prevent the adverse effects of UV radiation on skin, sunscreen cosmetics that include a UV radiation absorbing agent or UV radiation protection powder are used, and, in terms of antiperspiration, water-in-oil emulsified cosmetics are known to be suitable.

On the other hand, in recent years, skin care agents, such as creams, essences, and emulsions, that have a high sun screening effect are desired and used. Therefore, there is a growing demand for a base agent that contains a high blend ratio of a drug that has an ultraviolet protection effect and yet shows good usability such as smoothness and good spreadability.

Conventionally known formulations containing a drug having an ultraviolet protection effect include a water-in-oil emulsified cosmetic containing a cinnamic acid type ultraviolet absorbent, oil components, surfactants, and water (Patent Document 1) and a water-in-oil emulsified cosmetic containing zinc oxide, volatile silicone, silicone surfactants having a specific structure, and water (Patent Document 2). However, these water-in-oil emulsified cosmetics all used a large amount of zinc oxide to achieve a high ultraviolet protection and there was a shortcoming in terms of usability such as squeakiness and filminess.

Also, a known example of prior art technology that blends in the benzophenone ultraviolet absorbent that is used in the present invention is Patent Document 3; however, this invention relates to the photostability and the ultraviolet absorption effect and does not mention usability.

### PRIOR ART DOCUMENTS

### PATENT DOCUMENTS

- Patent Document 1:: JP 2009-67683 A
- Patent Document 2:: JP 2005-232068 A
- Patent Document 3:: JP H9-235216 A

"Use of specific UV filter combinations comprising BBDAPT for cosmetic preparations", IP.COM JOURNAL, IP.COM INC., WEST HENRIETTA, NY, US, (20100618), ISSN 1533-0001, "W/Si Natural Foundation Veil" describes a water-in-oil emulsified cosmetic.

### DISCLOSURE OF INVENTION

### TECHNICAL PROBLEM

The object of the present invention is to provide a water-in-oil emulsified cosmetic that is superior in terms of the ultraviolet protection effect and usability.

### TECHNICAL SOLUTION

The inventors conducted earnest research and as a result discovered that a water-in-oil emulsified cosmetic with good usability that has a high ultraviolet absorption effect and yet is free of squeakiness and spreads well can be obtained by using 2-hydroxy-4-methoxybenzophenone and a specific volatile oil component.

The present invention is a water-in-oil emulsified cosmetic that characteristically comprises the following (a)-(d) wherein the total blend ratio of (a) and (b) is 7-40 wt%:
(a) Isododecane 3.0-30 wt%
(b) Dodecamethylcyclohexasiloxane 1.0-10 wt%
(c) Alkyl/polyether comodified silicone having branched siloxane bonds 0.1-5.0 wt%
(d) One or more ultraviolet absorbents selected from 2-hydroxy-4-methoxybenzophenone and diethylamino hydroxybenzoyl hexyl benzoate 0.1-5 wt%,
and wherein the blend ratio (mass ratio) of (a) and (b) is (b)/(a) = 0.2-0.8.

### ADVANTAGEOUS EFFECTS OF THE INVENTION

The water-in-oil emulsified cosmetic of the present invention has a high ultraviolet protection effect, is smooth, spreads well, and has good texture.

### BRIEF DESCRIPTION OF DRAWINGS

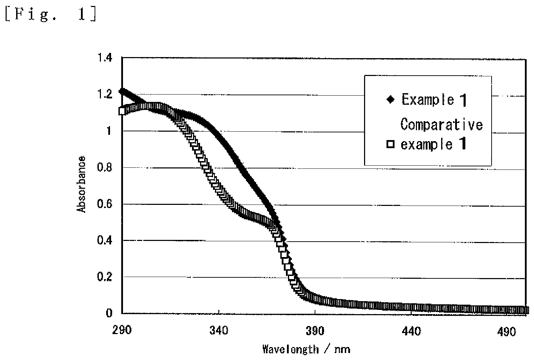

(FIG. 1) FIG. 1 is a drawing that shows the evaluation results for Example 1 and Comparative example 1

### BEST MODE FOR CARRYING OUT THE INVENTION

The present invention uses volatile oil components containing specific amounts of 2-hydroxy-4-methoxybenzophenone, isodecane, and dodecamethylcyclohexasiloxane to obtain a water-in-oil emulsified cosmetic with good usability that spreads smoothly while maintaining a high ultraviolet absorption effect.

The constituent ingredients are described in detail below.

### ((a) Isodecane, (b) Dodecamethylcyclohexasiloxane)

(a) isododecane and (b) dodecamethylcyclohexasiloxane used in the present invention are both volatile oil components, and usability worsens if either of them is missing.

The total blend ratio of (a) isododecane and (b) dodecamethylcyclohexasiloxane is 7-40 wt%, preferably 10-25 wt%, relative to the total amount of the emulsified cosmetic. The blend ratio of (a) isododecane is 3.0-30 wt%, preferably 5-15 wt%. The blend ratio of (b) dodecamethylcyclohexasiloxane is 1.0-10 wt%, preferably 2-8 wt%.

The usage ratio of (a) isododecane and (b) dodecamethylcyclohexasiloxane, or (b)/(a) (mass ratio), is (b)/(a) = 0.2-0.8, preferably (b)/(a) = 0.3-0.5.

Provided that the aforementioned requirements are met, volatile oil components other than isododecane and dodecamethylcyclohexasiloxane can be blended in; the blend ratio of all the volatile oil components is preferably 10-25 wt% relative to the total amount of the water-in-oil emulsified cosmetic. Of the volatile oil components, all may be isododecane and dodecamethylcyclohexasiloxane and it is preferable that 10 wt% or more is isododecane and dodecamethylcyclohexasiloxane.

### ((c) Alkyl/polyether comodified silicone having branched siloxane bonds)

Alkyl/polyether comodified silicone having branched siloxane bonds, which is the (c) ingredient of the present invention, is silicone main chains having, as side chains, alkyl chains, polyether chains, and silicone chains.

Commercially available examples of the (c) ingredient include lauryl PEG-9 polydimethylsiloxyethyl dimethicone (silicone KF-6038, manufactured by Shin-Etsu Chemical Co., Ltd.).

The blend ratio of the (c) ingredient is 0.1-5.0 wt%, preferably 0.3-3.0 wt%. Too much of the (c) ingredient causes usability to be sticky, and too little of it causes poor emulsification.

### ((d) Ultraviolet absorbent)

(d) The ultraviolet absorbent of the present invention is one or more selected from 2-hydroxy-4-methoxybenzophenone and diethylamino hydroxybenzoyl hexyl benzoate; particularly preferable is 2-hydroxy-4-methoxybenzophenone. A high ultraviolet protection can be achieved without compromising usability by using these ultraviolet absorbents in combination with the aforementioned volatile oil components (a) and (b).

Although it is possible to use ultraviolet absorbents other than (d) of the present invention, it is preferable to have 10 wt% or more of one or more selected from 2-hydroxy-4-methoxybenzophenone and diethylamino hydroxybenzoyl hexyl benzoate relative to the total amount of the ultraviolet absorbent.

The blend ratio of the (d) ingredient is 0.1-5 wt%, preferably 0.3-3 wt%. Too much of the (d) ingredient causes precipitation after emulsification, and too little lessens the ultraviolet protection effect.

Examples of the ultraviolet absorbents other than the (d) ingredient include cinnamic acid derivatives such as ethylhexyl methoxycinnamate, isopropyl methoxycinnamate, and isoamyl methoxycinnamate; para-aminobenzoic acid (hereafter abbreviated as PABA) derivatives such as PABA, ethyl PABA, ethyl-dihydroxypropyl PABA, ethylhexyl-dimethyl PABA, and glyceryl PABA; salicylic acid derivatives such as homosalate, ethylhexyl salicylate, dipropylene glycol salicylate, and TEA salicylate; benzophenone derivatives such as benzophenone-1, benzophenone-2, benzophenone-3 or oxybenzone, benzophenone-4, benzophenone-5, benzophenone-6, benzophenone-8, benzophenone-9, and benzophenone-12; benzylidene camphor derivatives such as 3-benzylidene camphor, 4-methylbenzylidene camphor, and polyacrylamide methylbenzylidene camphor; triazine derivatives such as anisotriazine, ethylhexyl triazone, diethylhexyl butamido triazone, and 2,4,6-tris (diisobutyl-4'-aminobenzalmalonate)-s-triazine; phenylbenzimidazole derivatives such as disodium phenyldibenzimidazole tetrasulfonate; imidazole derivatives such as 4-imidazole acrylic acid ethyl ester and 5-methyl-2 phenylbenzimidazole; phenylbenzotriazole derivatives such as drometrizole, trisiloxane and methylenebis (benzotriazoryl tetrabutylphenol); anthranyl derivatives such as mentyl anthranilate; imidazoline derivatives such as ethylhexyldimethoxybenzylidene dioxoimidazoline propionate; benzalmalonate derivatives such as polyorganosiloxane having benzalmalonate functional groups; 4,4-diarylbutadiene derivatives such as 1,1-dicaroboxy (2,2'-dimethylpropyl)-4,4-diphenylbutadiene; phenylbenzimidazole-5-sulfonic acid and salts thereof; phenylene-bis-benzimidazole-tetrasulfonic acid and salts thereof; and octocrylene.

### ((e) Ultraviolet scattering agent)

In the present invention, the ultraviolet protection effect is increased even more by, in addition to the aforementioned essential ingredients (a)-(d), adding an ultraviolet scattering agent selected from hydrophobicized titanium dioxide and hydrophobicized zinc oxide.

The method of hydrophobicizing is not limited in particular; the treatment can be done with a prior art method. Examples include a treatment in which silicones such as methylhydrogen polysiloxane, methylhydrogen polysiloxane/dimethyl polysiloxane copolymer, and dimethyl polysiloxane are used, a treatment in which silane compounds such as octyltriethoxysilane and hexyltrimethoxysilane are used, a treatment in which a fatty acid such as palmitic acid or stearic acid is used, a metal soap treatment in which an alkali metal salt or alkali earth metal salt of said fatty acid is used, and a fluorine treatment in which diethanolamine perfluoroalkylphosphate, perfluoroalkyltrimethoxysilane, etc. are used.

Examples of the hydrophobicized titanium dioxide include commercial products such as "TTO-S-4" , "TTO-V-4" (both from Ishihara Sangyo Kaisha, Ltd.), "MT-100TV", and "MT-500SAS" (both from Teika Pharmaceutical Co., Ltd.); examples of the hydrophobicized zinc oxide include commercial products such as "MZ-505S" (from Teika Pharmaceutical Co., Ltd.) and "Z-Cote HP-1" (from BASF); they can be used preferably.

For the (e) ingredient, titanium dioxide is preferably those having an average primary particle size of 5-30 nm. As for the zinc oxide, those having an average primary particle size of 10-100 nm are used preferably.

The blend ratio of the (e) ingredient in the present invention is 2-15 wt%, preferably 2-10 wt%.

In addition to the aforementioned ingredients, other ingredients usually used in cosmetics can be added as necessary into the water-in-oil emulsified cosmetic of the present invention as long as the object and/or effect of the present invention is not adversely affected. Examples of such ingredients include water soluble polymers, oil soluble polymers, polymer powder, emulsifiers, waxes, alcohols, liquid fats and oils, ester oils, hydrocarbon oils, fatty acids, higher alcohols, fatty acid esters, various drugs, and organic modified clay minerals. However, the examples are not limited to those shown.

Formulation forms of the water-in-oil emulsified cosmetic of the present invention include those that are cream-like and emulsion-like (including the two-layer separation type). Specific applications of the cosmetics include sunscreens and primers.

### EXAMPLES

The present invention is described in detail below by referring to Examples, but the present invention is not limited to these Examples. Unless otherwise noted, the blend ratio is in wt%.

Before the description of Examples, the efficacy test method used in the present invention is described.

### (1) Evaluation of usability

Each formulation was evaluated for its usability with actual use tests by a 10-member panel. When each emulsified cosmetic was applied, the "light spreadability" was evaluated using the following criteria.
⊚: 8 or more out of 10 reported "good."
○: 5-7 out of 10 reported "good."
Δ: 3-4 out of 10 reported "good."
×: 2 or less out of 10 reported "good."

### (2) Evaluation of stability

The emulsified cosmetics were put into a glass bottle and left alone for two weeks at 0° C, room temperature, and 50° C; the stability was evaluated using the following criteria:
○: No separation was observed.
Δ; Separation was observed only at low temperatures.
×: Separation was observed at each temperature.

### "Examples 1-11, wherein Examples 8 and 9 are Reference examples, Comparative examples 1-8"

Using the formulations shown in the following Tables 1-3, creams (water-in-oil type) were prepared and usabil.ity and stability were evaluated based on the aforementioned criteria. The results are also shown in Tables 1-3.

**{Table 1}**

| Ingredient names | | Example 1 | Comparative example 1 | Comparative example 2 | Comparative example 3 | Comparative example 4 | Comparative example 5 |
|---|---|---|---|---|---|---|---|
| | Ethanol | 5 | 5 | 5 | 5 | 5 | 5 |
| | Glycerin | 5 | 5 | 5 | 5 | 5 | 5 |
| | 1,3-butylene glycol | 5 | 5 | 5 | 5 | 5 | 5 |
| | Phenoxy ethanol | 0.5 | 0.5 | 0.5 | 0.5 | 0.5 | 0.5 |
| | Organic modified clay mineral *5 | 2 | 2 | 2 | 2 | 2 | 2 |
| (c) | polyether-modified silicone *1 | 3.5 | 3.5 | 3.5 | 3.5 | 3.5 | 3.5 |
| | Isostearic acid | 0.5 | 0.5 | 0.5 | 0.5 | 0.5 | 0.5 |
| | Pentaerythrityl tetraethylhexanoate | 5 | 5 | 5 | 5 | 5 | 5 |
| (b) | Dodecamethylcyclohexasiloxa ne | 4 | 4 | - | 14 | - | - |
| | Decamethylcyclopentasiloxan e | - | - | - | - | 14 | - |
| | Dimethylpolysiloxane *6 | - | - | - | - | - | 14 |
| (a) | Isododecane | 10 | 10 | 14 | - | - | - |
| | Octylmethoxy cinnamate | - | 2 | - | - | - | - |
| | Octocrylene | 5 | 5 | 5 | 5 | 5 | 5 |
| (d) | 2-hydroxy-4-methoxybenzophenone | 3 | - | 3 | - 3 | 3 | 3 |
| (e) | Hydrophobicized zinc oxide | 7 | 7 | 7 | 7 | 7 | 7 |
| | Cross-linked PMMA spherical powder | 5 | 5 | 5 | 5 | 5 | 5 |
| | EDTA-3Na·2H20 | 0.2 | 0.2 | 0.2 | 0.2 | 0.2 | 0.2 |
| | Purified water | Balance | Balance | Balance | Balance | Balance | Balance |
| Usability (light spredability) | | ⊚ | Δ | Δ | × | × | × |
| Stability | | ○ | ○ | ○ | ○ | ○ | ○ |

**{Table 2}**

| Ingredient names | | Example 2 | Comparative example 6 | Comparative example 7 |
|---|---|---|---|---|
| | Glycerin | 3 | 3 | 3 |
| | 1,3-butylene glycol | 7 | 7 | 7 |
| | Xylitol | 1 | 1 | 1 |
| | Organic modified clay mineral *5 | 2.5 | 2.5 | 2.5 |
| (c) | Polyether-modified silicone *1 | 3.5 | - | - |
| | Polyether-modified silicone *2 | - | 3.5 | - |
| | Polyether-modified silicone *3 | - | - | 3. 5 |
| | Dimethicone *4 | 5 | 5 | 5 |
| (b) | Dodecamethylcyclohexasiloxane | 4 | 4 | 4 |
| | Trimethylhexanoin | 5 | 5 | 5 |
| | Isostearic acid | 0.3 | 0.3 | 0.3 |
| (a) | Isododecane | 8 | 8 | 8 |
| | Polyoxybutylene polyoxypropylene glycol | 0.1 | 0.1 | 0.1 |
| | Octylmethoxy cinnamate | 5 | 5 | 5 |
| | Octocrylene | 5 | 5 | 5 |
| (d) | 2-hydroxy-4-methoxybenzophenone | 2 | 2 | 2 |
| (e) | Hydrophobicized zinc oxide | 4 | 4 | 4 |
| | Cross-linked PMMA spherical powder | 5 | 5 | 5 |
| | EDTA-3Na·2H20 | 0.2 | 0.2 | 0.2 |
| | Phenoxy ethanol | 0.5 | 0.5 | 0.5 |
| | Purified water | 38.9 | 38.9 | 38.9 |
| Stability | | ○ | Δ | × |

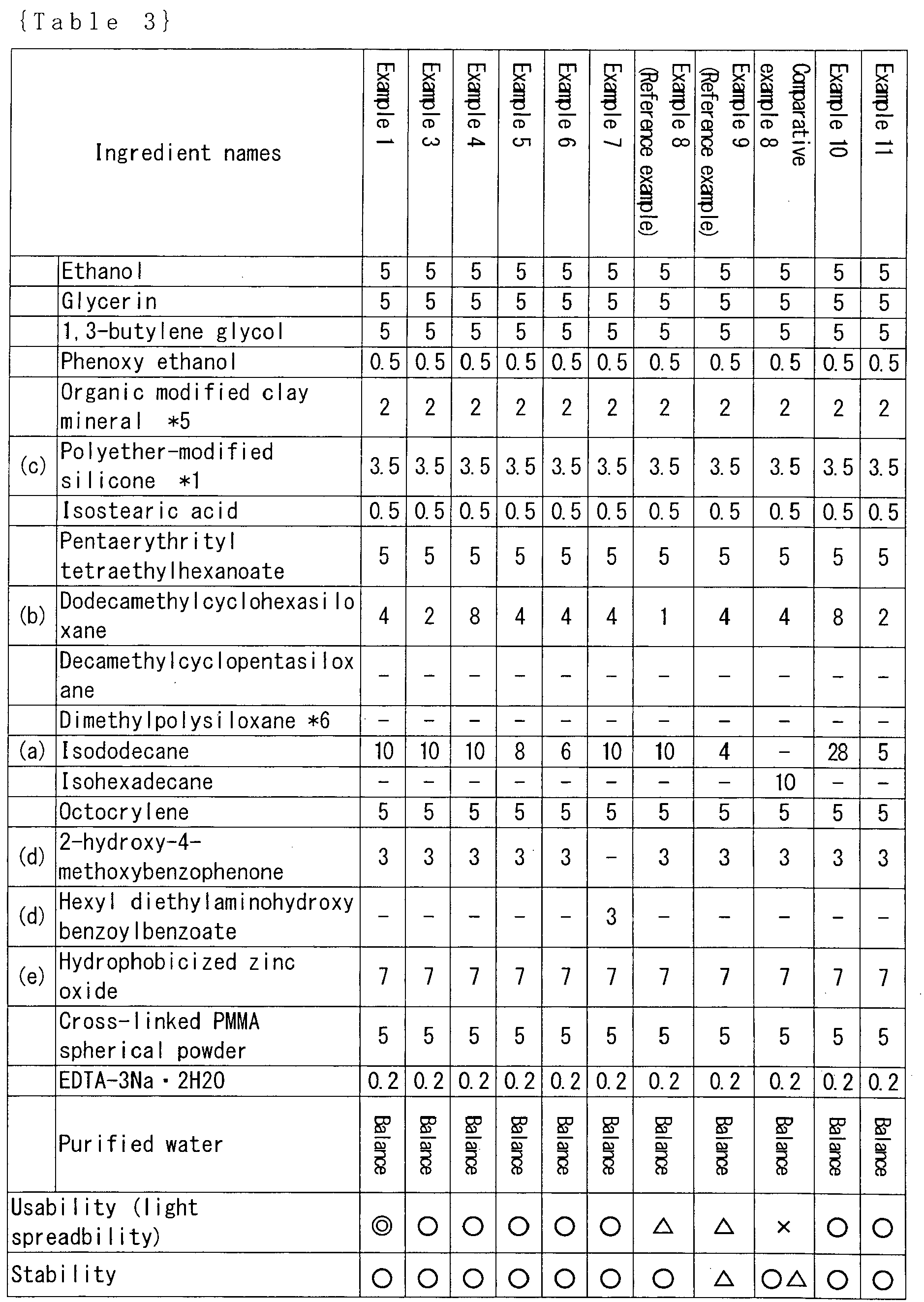

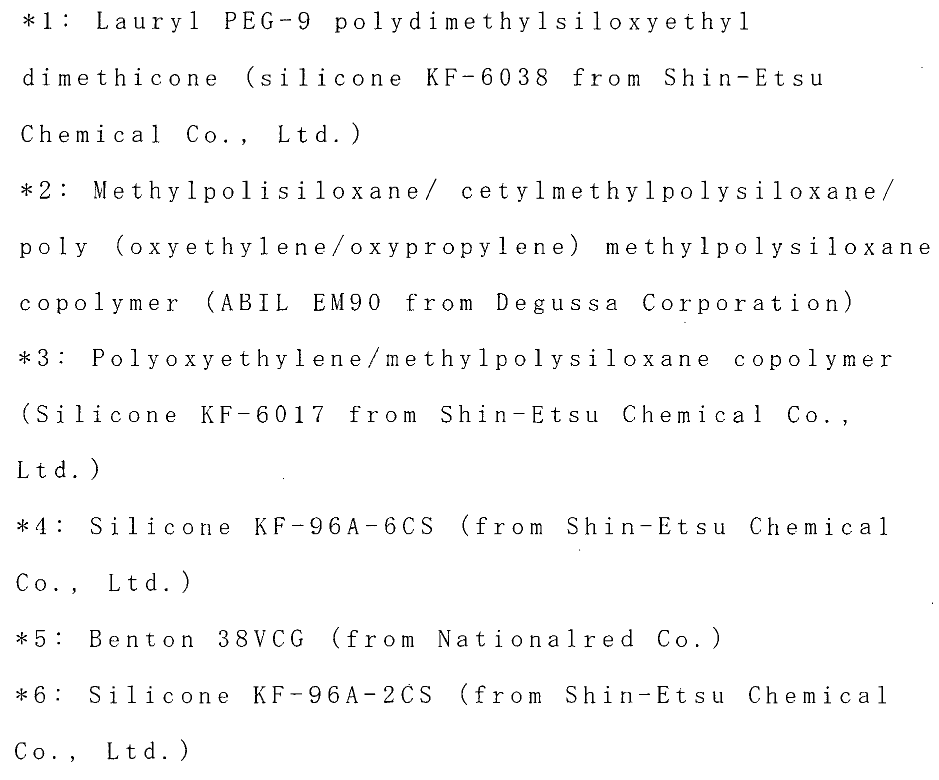

The emulsified cosmetics of Example 1 and Comparative example 1 were compared for ultraviolet protection in vitro and the evaluation was conducted with the following method. The results are shown in FIG. 1. FIG. 1 indicates that both Example 1 and Comparative example 1 have equivalent ultraviolet protection.

Other Examples and Comparative examples also gave results similar to FIG. 1.

### <Evaluation method>

A sample prepared using each formulation was applied on a PMMA plate in the amount of 0.75 mg/cm². The absorbance in the range of 290-500 nm was measured by using a spectrophotometer and a spectrum comparison was conducted.

Comparative example 1 contains octyl methoxycinnamate in place of 2-hydroxy-4-methoxybenzophenone. However, although this formulation shows suitable ultraviolet protection, the octyl methoxycinnamate, which is an oil component that is fluid at room temperature, affects usability of the product, which results in inferiority in terms of light spreadability as described in Table 1. The present invention can achieve a high ultraviolet protection by blending in 2-hydroxy-4-methoxybenzophenone without sacrificing usability.

Also, Comparative examples 2-5 are each constituted with a single volatile oil component (isododecane, dodecamethylcyclohexasiloxane, decamethylcyclopentasiloxane, and dimethylpolysiloxane) and each of them resulted in inferior usability. In contrast, Example 1 uses a combination of isododecane and dodecamethylcyclohexasiloxane to achieve good usability.

Formulation examples of the water-in-oil emulsified cosmetic of the present invention are shown below. Needless to say, the present invention is not limited at all by these formulation examples and it is specified by the scope of the claim.

### Formulation example 1

### (Water-in-oil type sunscreen cosmetic)

| Ingredient | wt% |
|---|---|
| (1) Ethanol | 5 |
| (2) Glycerin | 5 |
| (3) 1,3-butylene glycol | 5 |
| (4) Phenoxy ethanol | 0.5 |
| (5) Montmorillonite treated with benzyl dimethyl stearyl ammonium chloride (Product name: Benton 38VCG from Nationalred Co.) | 2 |
| (6) Polyether-modified silicone (Product name: KF6038 from Shin-Etsu Chemical Co., Ltd.) | 3.5 |
| (7) Isostearic acid | 0.5 |
| (8) Pentaerythrityl tetraethylhexanoate | 5 |
| (9) Decamethylcyclohexasiloxane | 4 |
| (10) Isododecane | 10 |
| (11) Octocrylene | 5 |
| (12) 2-hydroxy-4-methoxybenzophenone | 3 |
| (13) Hydrophobicized zinc oxide | 7 |
| (14) Cross-linked PMMA spherical powder | 5 |
| (15) EDTA-3Na 2H₂O | 0.2 |
| (16) Purified water | Balance |

### Preparation method:

(12) is heated and dissolved at 60 ° C in (11), to which (6)-(10) are added (oil phase). (3) is wetted with (4) and, together with (1)-(4), mixed with (16) in which (15) is already dissolved (water phase). (5) is added to the oil phase and dispersed with a disper, and then (13) is similarly dispersed in the oil phase with a disper. Finally, the oil phase and the water phase were mixed and emulsified with an emulsifier.

## Claims

1. A water-in-oil emulsified cosmetic that characteristically comprises the following (a)-(d) wherein the total blend ratio of (a) and (b) is 7-40 wt%:
(a) Isododecane 3.0-30 wt%
(b) Dodecamethylcyclohexasiloxane 1.0-10 wt%
(c) Alkyl/polyether comodified silicone having branched siloxane bonds 0.1-5.0 wt%
(d) One or more ultraviolet absorbents selected from 2-hydroxy-4-methoxybenzophenone and diethylamino hydroxybenzoyl hexyl benzoate 0.1-5 wt%, and wherein the blend ratio (mass ratio) of (a) and (b) is (b)/(a) = 0.2-0.8.

2. The water-in-oil emulsified cosmetic of claim 1 further comprising 2-15 wt% of an ultraviolet scattering agent selected from (e) hydrophobicized titanium dioxide and hydrophobicized zinc oxide.

## Patentansprüche

1. Wasser-in-Öl-emulgiertes Kosmetikum, das charakteristischerweise die folgenden (a)-(d) umfasst, wobei das Gesamtmischungsverhältnis von (a) und (b) 7 - 40 Gew.-% beträgt;
(a) Isododecan 3,0 - 30 Gew.-%
(b) Dodecamethylcyclohexasiloxan 1,0 - 10 Gew.-%
(c) Alkyl/Polyether-komodifiziertes Silikon mit verzweigten Siloxanbindungen 0,1 - 5,0 Gew.-%
(d) ein oder mehrere Ultraviolett-Absorptionsmittel, gewählt aus 2-Hydroxy-4-methoxybenzo-phenon und Diethylaminohydroxybenzoylhexylbenzoat 0,1 - 5 Gew.-%,
und wobei das Mischungsverhältnis (Massenverhältnis) von (a) und (b) als (b)/(a) = 0,2 - 0,8.

2. Wasser-in-Öl-emulgiertes Kosmetikum nach Anspruch 1, weiterhin umfassend 2 - 15 Gew.-% eines Ultraviolett-Streuungsmittels, gewählt aus (e) hydrophobisiertem Titandioxid und hydrophobisiertem Zinkoxid.

## Revendications

1. Un cosmétique émulsifié à l'eau dans l'huile qui comprend de manière caractéristique les éléments (a) - (d) suivants dans lesquels le rapport de mélange total de (a) et (b) est de 7 à 40% en poids:
(a) isododécane de 3,0 à 30% en poids,
(b) dodécaméthylcyclohexasiloxane de 1,0 à 10% en poids,
(c) silicone comodifiée à l'alkyle / au polyéther ayant des liaisons siloxane ramifiées de 0,1 à 5,0% en poids,
(d) un ou plusieurs absorbants ultraviolets choisis parmi la 2-hydroxy-4-méthoxybenzophénone et le benzoate de diéthylamino-hydroxybenzoyl-hexyle de 0,1 à 5% en poids, et dans lequel le rapport de mélange (rapport de masse) de (a) et (b) est (b) / (a) = 0,2 à 0,8.

2. Le cosmétique émulsifié à l'eau dans l'huile selon la revendication 1, comprenant en outre de 2 à 15% en poids d'un agent de diffusion ultraviolet choisi parmi (e) du dioxyde de titane hydrophobisé et de l'oxyde de zinc hydrophobisé.
